# EUROPEAN PATENT SPECIFICATION

(11) **EP 4 033 957 B1**
(45) Date of publication and mention of the grant of the patent: **19.07.2023**
(21) Application number: 20774998.7
(22) Date of filing: 23.09.2020
(51) Int. Cl.: A61B 1/247, A61B 1/32, A61B 1/00, A61B 1/04, A61B 1/06

(54) **LIP AND CHEEK RETRACTOR, SYSTEM AND METHOD FOR OBTAINING AN IMAGE OF A DENTAL ARCH**
LIPPEN- UND WANGENRETRAKTOR, SYSTEM UND VERFAHREN ZUM ERHALTEN EINES BILDES EINES ZAHNBOGENS
ÉCARTEUR DE LÈVRES ET DE JOUES, SYSTÈME ET PROCÉDÉ D'OBTENTION D'UNE IMAGE D'ARCADE DENTAIRE

(30) Priority: 25.09.2019 FR 1910606
(43) Date of publication of application: 03.08.2022
(73) Proprietor: Vigident, 6041 Charleroi (BE)
(72) Inventor: BENOLIEL, Simon, 75011 Paris (FR)
(74) Representative: Calysta NV
(86) International application number: PCT/EP2020/076595
(87) International publication number: WO 2021/058582

(56) References cited:
- CN-A- 106 473 693
- US-A- 836 967
- US-A1- 2012 003 602

## Description

### Technical Field

The present invention relates to a lip and cheek retractor and a system and method using such a retractor for obtaining an image of a dental arch of a patient.

### Prior Art

In the practice of dentistry, it is known to keep the lips and cheeks of a patient at a distance from his teeth in order to better examine the condition of the teeth or mucous membranes or in order to take a photographic picture of the entire dental arch, either in the upper or lower jaw. In fact, an oral examination concerns the examination of the inside of the mouth and includes the inspection of the upper and lower teeth on all their visible faces; vestibular, lingual or palatal and on their triturating or incisal surfaces. The oral examination also covers the mucous membranes; that of the palate, the tongue, the gums on their vestibular, lingual or palatal surfaces and the mucous membrane covering the inside of the cheeks and lips.

The other area of dentistry in which lip and/or cheek retractors are used concerns the protection of the patient's mucous membranes during a dental procedure; in particular during surgical operations, when using bleaching products or when performing procedures that must be carried out without saliva such as composite fillings or bonding veneers. One of the most common types of cheek retractor is called "Bishop Retractor". It is a surgical instrument grasped by a surgeon who holds the patient's cheek with one hand while performing surgery with the other hand.

Other retractors such as EP1844728, WO-A-96/29952 or WO-A-00/42939 will retract the lips and/or cheeks simultaneously on the left and right side of the patient.

More recently FR3065363 describes a complex imaging device with lip retractors incorporated into a case with a mirror system, which allows the position of the teeth to be measured and reproduced, in particular to follow the progress of a patient's orthodontic treatment. For this reason, FR3065363 only inspects and photographs the vestibular surfaces of the teeth, as the system is not designed to enter the mouth.

All of the retractors mentioned, or those found on the market, are designed according to the same principle: using the "natural" elasticity of the lips to make a bilateral lip retractor "stand alone", or having a unilateral lip retractor held by the patient or an operator. In the geriatric dental environment, patients are by definition very old and fragile. One of the effects of aging on the skin and mucous membranes is their loss of elasticity and dryness. For these patients, the use of a conventional lip retractor does not always seem to be appropriate, especially since the patient's cooperation is not always assured.

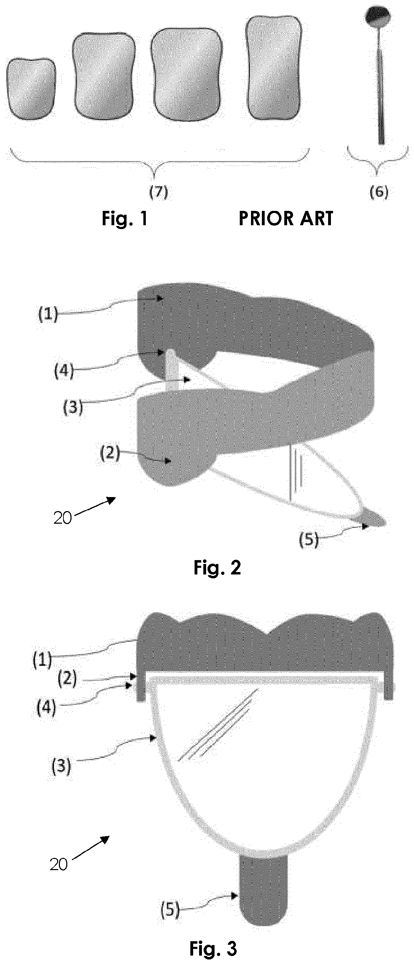

To take a picture of a dental arch, a photographic intraoral mirror 7 as shown in Fig. 1 and as sold on the dental market is used. The intraoral mirror 7 for dental photography differs from the oral examination mirror 6 known as the "dentist's mirror" by its size and shape. The classic dentist's examination mirror 6 serves as a mirror but also as a retractor when performing daily therapeutic procedures. In Ahmad I's state-of-the-art description of dental photography ("digital dental photography part 8, intra-oral set-ups"), he uses a bilateral lip retractor and intraoral mirrors of different shapes depending on the desired image. In his method, it is the patient whose teeth are to be photographed who can hold the intra-oral mirror 7. In other methods it is a Dental assistant operator who rotates and holds the mirror 7 while the "photographer" takes the picture. The interest of a photographic intraoral mirror 7, which extends over an area equivalent to that of a whole dental arch, is therefore the possibility of "seeing" and possibly recording the whole dental arch on a single photographic photograph. Hence the various intraoral photographic techniques described in particular by the reference in the matter; Ahmed I. The intra-oral mirrors for taking a photographic photograph are medical devices that can be disinfected and sterilized.

Thus, the state-of-the-art mirrors for taking photographs of the dental art are cumbersome. It requires one hand for the retractor, one hand for the mirror and one hand for making the photo. Therefore, it requires always at least two persons to take the photo. In addition, the images of the same dental arc taken at two different time instances are not comparable as the holding angles of the mirror in the mouth are different and are not known.

There are clamps or retractors with reflective surfaces for improving the visibility of certain surfaces of the dental arc for a surgery or for taking a photo like in US836967, US9610009, US2012/0003602 or US1157565. However, those clamps or retractors do either obscure certain surfaces of the dental arc or show only a part of the dental arc. Prior art document US836967 discloses a dental mirror comprising a frame comprising in its rear part on the right and on the left side a support and a mirror being rotatable in the supports of the frame. US836967 also discloses a dental mirror comprising a frame comprising in its rear part on the right and on the left side a support and an intermediate part being rotatable in the supports of the frame and being configured to fix a mirror.

There are further known retractors for making photographs of the dental arch like in EP3415082. However, the retractor uses the elasticity of the cheeks and lips to improve the visibility of the mouth which is not well suitable for elderly persons. The system discloses a mirror placed outside of the mouth to get several different views of the dental arch to create a 3D view of the dental arch. The system does however not show the full dental arch and not all sides of the dental arch.

### Summary of the invention

It is the object of the present invention to improve the way of taking photos of the dental arc of patients.

The invention is defined in the appended claims.

In the embodiment defined in independent claim 1 a lip and cheek retractor for examining the oral cavity of a person, comprising a U-shaped frame which follows the form of a dental arch and comprising in its rear part on the right and on the left side a support and a mirror being rotatable in the supports of the frame, wherein the frame is configured to be placed in the facial side space between a dental arch, a lip and cheeks of the oral cavity of the person such that the frame follows the anatomy of a vestibular alveolar bone of the person is provided.

In the embodiment defined in independent claim 7 a lip and cheek retractor for examining the oral cavity of a person, comprising a U-shaped frame which follows the form of a dental arch and comprising in its rear part on the right and on the left side a support and an intermediate part being rotatable in the supports of the frame and being configured to fix a mirror, wherein the frame is configured to be placed in the facial side space between a dental arch, a lip and cheeks of the oral cavity of the person such that the frame follows the anatomy of a vestibular alveolar bone of the person is provided.

Some preferred embodiments relate to a system for taking a photographic image of a dental arch of a person comprising said retractor and a camera for taking a photographic image of the mirror showing the dental arch of the person, when the retractor is placed in the oral cavity of the person.

Optionally, the system further comprises a processing means for analysing the dental arch of the person based on the photographic image.

Further embodiments of the claimed invention relate to a method for taking a photographic image of a dental arch of a person comprising the steps: placing said retractor into the oral cavity of the person in an examination position; and using a camera to take a photographic image of the mirror showing the dental arch of the person, when the retractor is placed in the examination position.

Such a method is defined in claim 14. Optionally, the method further comprises the steps: receiving, in a processing means, a photographic image of the dental arch of the person and analysing the dental arch of the person based on the photographic image, wherein the analysis of the dental arch comprises the detection of at least one health problem of the dental arch, wherein the at least one health problem is detected in the image based on an artificial intelligence engine of the processing means.

Subsequently, advantageous embodiments of the invention are described.

In one embodiment, the mirror is configured to show the dental arch, preferably with all teeth of the dental arch of the person (when the retractor is placed in the examination position).

According to the invention, the frame is configured to be placed in a well-defined examination position in the oral cavity of the person. The examination position is the vestibular space of the dental arch to be analysed. Since the retractor or frame is configured to be placed in the well-defined examination position, the position of the retractor in the mouth and thus the position of the mirror is reproducible. This improves the comparability of different images taken from the dental arch of the same person or of different persons as the mirror has a well-defined position with respect to the dental arch to be analysed. The well-defined position also improves the quality of the images of the dental arch taken with the retractor and facilitates the way of taking images of the dental arch.

In one embodiment, the frame and the mirror have an examination angle between 30° and 60°.

According to the invention, the frame has a U-shape form and is configured to be placed (in the examination position) in the vestibular space in other words is configured to be placed (in the examination position) along a facial side of the dental arch of the person.

According to the claimed invention, the mirror or the intermediate part is supported rotatably in the frame. Thus, the mirror can be moved/rotated between an examination angle and a rest angle. This reduces the size of the retractor in the rest position. The mirror or the intermediate part is supported rotatably in supports at the distal ends or rear parts of the frame. Therefore, the mirror can be rotated in the examination angle which opens towards the opening of the mouth so that a high-quality picture of the dental arch can be taken from a comfortable position outside of the mouth. Preferably, the rear parts or distal ends comprise each a protrusion in an active direction or an ear, wherein the supports are arranged in the protrusions or ears. This improves the distance of the mirror in the rear part of the mouth and increases the visibility of the molar teeth. Preferably, the mirror can be fixed in the examination angle. This allows to obtain a reproducible examination angle which improves the quality and comparability of the images. It further allows to hold the retractor only at the mirror or the frame without changing the examination angle, while inserting the retractor in the mouth. The examination angle is preferably between 30° and 60°. This range allows a comfortable camera position with respect to the mirror/mouth to take the photo of the dental arch reflected in the mirror.

In one embodiment, the U-shaped frame extends in its rear part on the right and on the left in an ear, in the centre of which is a support, wherein the mirror can rotate in the supports. Preferably, the mirror can rotate in the supports at the level of the ears.

In one embodiment, the supports at the distal/rear end of the frame are a material connection between the frame and the mirror configured to allow a rotation of the mirror in the supports of the frame. The support comprises preferably a bending line to bend the frame with respect to the mirror allowing such a rotation of the mirror around the bending line. This allows to manufacture the frame and the mirror out of one piece.

In one embodiment, the retractor, preferably the mirror comprises a gripping handle. This allows to insert the retractor in (the examination position in) the mouth of the person. Preferably, the gripping handle is arranged at the end of the mirror. The end of the mirror is preferably coming out of the mouth, when the retractor/frame is in the examination position.

In one embodiment, the mirror allows a view of an entire dental arch the person, i.e. of all teeth of the dental arch, and/or allows to obtain an image of the entire dental arch in a single view. Thus, one image is sufficient to analyse the dental arch and with two images, the complete oral cavity of the person can be analysed. The mirror allows to view the lingual side and the active side of the dental arch of the person. The facial side of the teeth of the dental arch could be seen directly and/or via a secondary mirror described below. Directly means here without a reflection in the mirror or the secondary mirror of the retractor.

In one embodiment, the frame/retractor comprises a secondary mirror arranged such that, when the frame/retractor is placed in the mouth in the examination position, a facial side of the dental arch is reflected via the secondary mirror on the mirror. This allows to see also the facial side of the teeth of the dental arch on the mirror. Thus, one single image of the mirror shows all three sides of the dental arch to be analysed. Preferably, all three sides of the dental arch are shown in one single plane of the mirror. By having all three sides of the dental arch shown in a reproducible way in the same plane of the mirror, the images are particular well suited for digitally analysing the image to retrieve conclusions about the dental arch. Preferably, the secondary mirror comprises a plurality of flat sub-mirrors, preferably five flat sub-mirrors. The flat sub-mirrors allow to reflect the image of the facial side of the dental arch without distortions from a curvature of the sub-mirror on the mirror which improves the quality of the image and facilitates the processing of the image. The plurality of sub-mirrors serves to reflect different portions of the dental arch on the mirror and/or to keep the different sub-mirrors substantially parallel to the interdental direction of the dental arch. Preferably, the secondary mirror is arranged on the lingual side of the frame. Preferably, the secondary mirror is realised as a reflective surface on the frame.

In one embodiment, the retractor comprises a lighting system to illuminate the oral cavity, when the retractor is placed in the examination position.

In one embodiment, the mirror is fixable on the intermediate part. This allows to use the retractor with commercially available mirror, like intro-oral mirrors. In one embodiment, the intermediate part comprises a cylinder and a rod. Said cylinder being preferably secured to the rod which passes through it over its entire length. Preferably, said rod exceeding in length that of the cylinder and thus forming rotation pins which are supported in the supports provided in the frame, the cylinder being configured to fix the mirror. In one embodiment, intermediate part or the cylinder comprises a groove running over the entire length of the intermediate part or the cylinder for fixing the mirror by friction. Preferably, the intermediate part or the cylinder comprises a further groove also running along the entire length of the intermediate part or the cylinder and whose width is greater than that of the groove. This allows to fix two different type of mirror in the intermediate part.

According to the claimed invention, the frame is U-shaped and follows the form of the dental arch.

In one embodiment, the mirror is supported at distal ends of the frame.

In one embodiment, the mirror is supported rotatably in the frame and can be rotated between an examination angle and a rest angle. Preferably, the mirror can be fixed in the examination angle. The rotatable support can be achieved by classic support of an axle in a support or by a bending line of the support which allows a rotation/bending of the frame with respect to the mirror. In the first case, the rest angle is preferably zero degree. In the second case, the rest angle is preferably 180°.

In one embodiment, the mirror and the frame is made out of one piece. In this case, the rotation of the mirror in the supports of the frame could be arranged by a bending line in the supports of the frame. The supports could unit to a common support structure so that the bending line in the supports could be also in the common support structure.

In one embodiment, the mirror and the frame are made of at least two pieces which are assembled together to provide the retractor.

In one embodiment, the retractor is configured to show the dental arch of the person in the mirror, when the retractor is placed in an examination position in an oral cavity of the person.

In one embodiment, the retractor comprising further a secondary mirror configured to reflect an image of a facial side of the dental arch on the mirror so that the mirror shows the lingual side, the active side and the facial side of the dental arch.

In one embodiment, the system comprises a retractor, a camera and a processing means.

In one embodiment, the processing means is preferably a server receiving via the internet the image of the dental arch taken by the camera.

In one embodiment, the image of the dental arch is taken without a retractor.

In one embodiment, the image of the dental arch is taken with a retractor for separating the lip and/or cheeks from the dental arch which does not comprise a mirror (a classic retractor).

In one embodiment, the image of the dental arch is taken with a retractor for separating the lip and/or cheeks from the dental arch with a mirror, preferably a retractor as described above. In this case, the camera takes an image of the mirror showing the dental arch of the person.

In one embodiment, a first image of the upper dental arch is taken, and a second image of the lower dental arch is taken. Preferably, the first image is taken with the retractor in a first examination position in the upper jaw and the second image is taken with the retractor in a second examination position in the lower jaw. Preferably, the processing means, the analysis section or the analysis step comprises analysing of the oral health state person based on the analysis of the upper dental arch based on the first image and based on the analysis of the lower dental arch based on the second image. This allows a full health assessment of the oral cavity of the person to be analysed.

In one embodiment, the analysis of the dental arch or of the oral cavity comprises the detection of at least one health problem of the dental arch or the oral cavity, preferably a plurality of health problems of the dental arch or the oral cavity. If the detection of the at least one health problem results that the dental arch or the oral cavity does not show any of the at least one health problem, the analysis results that the dental arch or the oral cavity is in a good health state. If the detection of the at least one health problem results that the dental arch or the oral cavity shows at least one of the at least one health problem, the analysis results that the dental arch or the oral cavity is not in a good health state and/or results in a recommendation what to do. The recommendation could be to visit a dentist urgently, to visit a dentist in a certain time period or simply to take a certain action. The certain action could be one or more of: use a certain type of toothbrush, use a certain type toothpaste, brush the teeth more frequently, use dental floss, make another analysis in a certain time period to take a further recommendation, etc.

In one embodiment, the at least one health problem comprises at least one health problem other than the position of the teeth in the dental arch or in the oral cavity. The at least one health problem comprises preferably one or more of at least one hard tissue problem and at least one soft tissue problem. The at least one hard tissue problem refer principally to at least one problems of the teeth of the dental arch(es). The at least one hard tissue problem comprise preferably one or more of dental caries, dental plaque, dental abscess, dental decay, dental erosion. The at least one soft tissue problem comprises gum health problems, palate health problems (for the upper dental arch), tongue health problems (for the lower dental arch), lip health problems, cheek health problems.

In one embodiment, the at least one health problem is detected in the image based on image recognition, e.g. based on the recognition of a certain colour, pattern, etc. of the dental arch.

In one embodiment, the image recognition or the analysis comprises the detection of the different parts of the dental arch in the different portions of the image. The different parts comprise preferably the different teeth of the dental arch, the gums of the dental arch, the tongue, and/or the palate. The different parts can be further sub-categorized. The different teeth comprise preferably the facial side of each tooth of the dental arch, the active side of each tooth of the dental arch and the lingual side of each tooth of the dental arch. The gums comprise preferably as different parts the gum of each tooth, even more preferably the facial side of the gum of each tooth and the lingual side of the gum of each tooth. The tongue and/or the palate can also be subdivided in different parts. Preferably, the at least one health problem is analysed for different ones of the different parts of the dental arch. Preferably, each one of the at least one health problem has at least one associated part of the dental arch in which the health problem can appear. Preferably, the at least one health problem is detected in each of the at least one associated part of the dental arch based on the at least one respective image portion showing the at least one associated part of the dental arch. Preferably, hard tissue health problems are analysed for the different parts of the teeth of the dental arch. Preferably, soft tissue health problems are analysed for the different parts of the soft tissue of the dental arch. Preferably, gum health problems are analysed for the at least one different part of the gums of the dental arch. Preferably, tongue health problems are analysed for the at least one different part of the tongue of the dental arch. Preferably, palate health problems are analysed for the at least one different part of the palate of the dental arch. When a certain health problem is detected in a certain part of the dental arch, the certain health problem is stored in relation to the certain part.

In one embodiment, the at least one health problem is detected in the image based an artificial intelligence engine. The artificial intelligence engine can be based on machine learning. The machine learning could be based on a decision tree or on deep learning or on another machine learning technique. The artificial intelligence engine could be based on a neuronal network. The artificial intelligence engine could be trained based on the at least one health problem identified or verified in a certain region of the image of the dental arch by a human, preferably a dentist. The artificial intelligence engine could be continuously trained by a feedback of a dentists confirming or non-confirming the at least one health problem detected by the processing means/analysis section and/or by a feedback from the dentist indicating further health problems. The conclusions from the dentist can be retrieved from the same images and/or from a visit of the patient at the dentist. Preferably, the artificial intelligence engine uses for training/detection a feature vector. The feature vector could be different for different health problems. The feature vector could comprise colour, pattern or form of certain features of the image, or of an image portion used for the detection of a health problem.

In one embodiment, the processing means could send the image(s) of the dental arch to a dentist for analysing. This can be done without analysing the image(s) in the processing means or in addition to the analysis of the image(s) in the processing means. The additional analysis by a dentist could be to verify the analysis of the processing means or to make another type of analysis.

In one embodiment, a first image the dental arch is taken at a first point in time, and a second image of the same dental arch is taken at a second point in time. The first and second point in time are preferably more distant than 1 hour, preferably than 1 day, preferably than 1 week, preferably than 1 month. Preferably, the processing means, the analysis section or the analysis step comprises comparing the first image and the second image. The comparison of the two images results in an evolution of the health state of the dental arch between the two points in time. The evolution of the health state of the dental arch could be used to detect certain health problems. Preferably, the evolution comprises health problems which are new and/or which disappeared from the first to the second image. Preferably, certain health problems could only be detected by a trend of a state of the dental arch between the two points in time. For example, a velocity of the reduction of the gums could only be detected from such an evolution between two points in time.

### Brief description of the Drawings

Figure 1 shows state of the art intraoral mirrors.
Figure 2 shows a schematic three-dimensional view of a first embodiment of the lip and cheek retractor according to the invention.
Figure 3 shows a schematic top view of the first embodiment of the retractor according to the invention.
Fig. 4 shows a schematic three-dimensional view of the fixation for a mirror for a second embodiment of the lip and cheek retractor according to the invention.
Fig. 5 shows a system with a retractor according to the invention for taking a photographic image of the dental arch of the patient.
Fig. 6 shows a method with a retractor according to the invention for taking a photographic image of the dental arch of the patient.
   In the drawings, the same reference numbers have been allocated to the same or analogue element.
Fig. 7 shows an embodiment of the frame with a secondary mirror.
Figure 8 shows a schematic three-dimensional view of a third embodiment of the lip and cheek retractor according to the invention.
Figure 9 shows a schematic top view of the third embodiment of the retractor according to the invention.

### Detailed description of an embodiment of the invention

Other characteristics and advantages of the present invention will be derived from the non-limitative following description, and by referring to the drawings and the examples.

The retractor, the system, and the method according to the invention are used for a human person, also called patient. The retractor is normally inserted in the mouth or oral cavity of the patient.

Before describing the retractor, the terms used in the mouth of the patient shall be defined. The mouth has two jaws, the maxillary or upper jaw and the mandibular or lower jaw. Each jaw comprises a dental arch. The dental arch (of each jaw) comprises a plurality of teeth, e.g. an adult comprises normally 16 teeth in each dental arch. The dental arch comprises a first quadrant and a second quadrant, each comprising one half of the dental arch. The dental arch and each of its teeth comprise three visible sides comprising a lingual side, a facial side and an active side. The lingual side faces towards the interior of the mouth or towards the tongue of the patient (at least in a horizontal direction). The lingual side can also be called the palatal side. A lingual direction is correspondingly a direction pointing in the plane of the dental arch into the interior of the mouth. The facial side faces to the exterior of the mouth or of the dental arch or faces towards a lip or cheek of the patient. The facial side of the teeth facing towards the lip (central and lateral incisors and canines) is also called labial side. The lip refers always to the lip corresponding to the respective dental arch. If the upper dental arch is meant, the upper lip is meant. If the lower dental arch is meant, the lower lip is meant. The facial side of the teeth facing towards one of the cheeks (premolars and molars) is also called buccal side. The facial side can also be called the vestibular side. A facial direction is thus a direction in the plane of the dental arch pointing towards the lip or cheek of the patient. The facial side/direction and the lingual side/direction on the same point of the dental arch or for the same tooth are opposed to each other. The active side of the teeth refers to the incisal sides (for the incisors) and to the triturating (for the premolars and molars) sides of the teeth. The active direction is thus perpendicular to the plane of the dental arch or to the facial and/or lingual direction. The dental arch and each of its teeth comprise further three non-visible sides comprising two interdental sides and one root side. The interdental sides refer to the sides between two neighbouring teeth. The interdental sides facing towards the back of the mouth are called distal sides or rear sides. Thus, a distal direction extends along the dental arch from the zenith of the dental arch or the incisors towards the back of the mouth or the molars. The two ends of the dental arch in the distal direction are called the two distal ends of the dental arch. The interdental sides facing towards the zenith of the dental arch are called the mesial side. Thus, a mesial direction extends along the dental arch from the back of the mouth or the molars or the distal ends towards the zenith of the dental arch or the incisors. The mesial direction and the distal direction on the same point of the dental arch or for the same tooth are opposed to each other (substantially 180°). The mesial direction and/orthe mesial direction are parallel to the plane of the dental arch. The root side faces the roots of the teeth. The root direction faces from the dental arch or its teeth towards its/their roots or towards gums in which the dental arch is hold. The root direction/side is opposed to the active direction/side. The root direction is perpendicular to the plane of the dental arch. The first and second quadrants are separated by a so-called median line. The median line corresponds also to the symmetry line of the dental arch. The median line defines the median direction. The median line/direction is arranged in the plane of the dental arch and/or is perpendicular to the active or root direction. The median line/direction extends from the throat to the mouth opening or vice versa. The throat direction extends along the median direction towards the throat. The opening direction extends along the median direction towards the mouth opening. The definition of these sides or directions apply equally for other objects placed in the mouth or other parts of the mouth, if their sides have substantially the same surface normal as the corresponding sides of the dental arch or its teeth. The vestibular space or the facial space shall refer herein to the space between the teeth of the dental arch and the lip and the cheeks. The vestibular space has a first buccal space, a second buccal space and a labial space. The first buccal space is between a first cheek and the teeth of the first quadrant of the dental arch. The second buccal space is between a second cheek and the teeth of the second quadrant of the dental arch. The labial space is between a lip and the teeth of the dental arch. The definition of the sides and directions apply equally for the upper and the lower dental arch. The only difference is that the root direction/side and the active side/direction are inverted, when changing from one of the two dental arches to the other one. The term mouth in here is used equivalent with the term oral cavity.

Fig. 2 and 3 show a first embodiment of retractor 20 according to the invention. The retractor 20 comprises a frame 1 and a mirror 3.

The frame 1 is configured to be placed into the mouth of the patient. The frame 1 is placed in a well-defined position in the mouth of the patient. This well-defined position is herein also called the examination position. The frame 1 is configured/designed to be placed in this examination position. The examination position is in the vestibular space. The frame 1 in the examination position extends along the dental arch in the vestibular space, preferably from the first buccal space (over the labial space) to the second buccal space. Preferably, the frame 1 extends from the first distal end of the dental arch to the second distal end of the dental arch. This has the advantage that the cheeks and the lip is hold away from all/the teeth of the dental arch and improves the visibility of the facial side of the teeth and/or gums of the dental arch. The frame 1 in the examination position extends along the vestibular side of the alveolar bone (also called alveolar process) of the dental arch. The frame 1 butts/touches the facial or vestibular side of the dental arch, preferably of the alveolar bone of the dental arch. This allows to obtain a reproduceable examination position as the dental arch or the alveolar bone has always the same position. The frame touching/butting the alveolar bone obviously means that the frame 1 touches/butts the gums coving the alveolar bone. The frame 1 is preferably designed such that it does not cover/touch the teeth of the dental arch. This can be achieved by reducing the height of the frame 1 so that it touches only the gums, but not the teeth of the dental arch. The frame 1 is preferably designed such that the lip and/or the cheeks are pushed away/separated/kept apart from the dental arch, when the frame 1 is placed in the examination position. This allows that the facial side of the teeth and the gums of the dental arch are well visible and not covered by the lip and cheeks. The frame 1 is preferably designed such that a repeated insertion of the frame 1 in the same dental arch of the same patient would lead to the same examination position of the frame 1 with respect to the dental arch. The frame 1 has a U-shaped form which corresponds to the U-shaped form of the dental arch. The U-shape of the frame 1 defines a frame-plane which is parallel to the plane of the dental arch, when the frame 1 is placed in the examination position. The frame 1 has preferably a lingual side, a root side and a facial side. The lingual side of the frame 1 faces in the examination position the facial side of the dental arch. The facial side of the frame 1 faces in the examination position the lip and/or cheeks. The root side faces in the root direction. Preferably, the lingual side of the frame 1 touches the facial side of the gums and/or the dental arch (at least in some points). This allows to define the examination position in a reproduceable way. Preferably, the facial side of the frame 1 touches the lip and/or cheeks of the patient. The width and/or form of the frame 1 is preferably such that the lip and/or cheek is hold away from the dental arch of the patient. The width of the dental arch is the distance between the facial side and the lingual side of the frame 1.

The mirror 3 has a reflective side, a back side and at least one peripheral side. The reflective side is configured to show the dental arch, when the retractor 20 is placed in the examination position (and the mirror 3 is in the examination state). The reflective side of the mirror 3 faces preferably towards the frame 1 or in the examination position towards the dental arch. The back side is opposed to the reflective side. The at least one peripheral side comprises preferably a throat side and a peripheral side. The throat side is arranged in the throat direction of the mirror 3. The throat side is preferably substantially straight. The throat side could however be formed differently as well. The peripheral side refers to the remaining peripheral side(s) of the mirror 3. Preferably, the peripheral side has a U-shape form and/or the form of the frame 1. Preferably, the peripheral side of the mirror 3 follows the form of the frame 1, thus of the dental arch of the patient.

The mirror 3 is supported in the frame 1. This allows to have a reproduceable mirror position in the mouth when the frame 1 is placed in the examination position in the mouth of the same patient. The mirror 3 (in an examination state of the retractor 20) is configured to make the dental arch, preferably the entire dental arch of the patient visible from the exterior of the mouth. The mirror 3 (in the examination state) is preferably configured to show (on its reflective side) the lingual side and the active side of the dental arch, i.e. of the teeth and/or gums of the dental arch (from a viewpoint outside of the mouth). The viewpoint is preferably at the height of the mouth opening. The mirror 3 in an examination state has a pre-defined examination angle with respect to the plane of the frame 1 or, when placed in the examination position, with respect to the plane of the dental arch. The mirror 3 is preferably supported at the distal ends of the frame 1. This causes the mirror 3 in the examination state/angle to open towards the mouth opening or towards the lips. The examination angle is preferably larger than 30°, preferably larger than 35°, preferably larger than 40°, preferably larger than 42°. The examination angle is preferably smaller than 60°, preferably smaller than 55°, preferably smaller than 50°, preferably smaller than 48°. The examination angle is preferably between 30° and 60°, preferably between 35° and 55°, preferably between 40° and 50°, preferably between 42° and 48°. The examination angle is preferably 45°. The mirror plane and the frame plane in the examination state/angle intersects preferably at the throat side of the mirror 3 and/or at the distal ends of the frame 1. The mirror 3 is preferably composed by a single mirror. However, it is also possible that the mirror 3 comprises multiple sub-mirrors constituting together the mirror 3 described in here. In this case, the sub-mirrors are arranged preferably in the same mirror plane. However, it would also be possible to arrange the sub-mirrors in different mirror planes which would however be a bit more complicated to analyse the images of the dental arch. In this case, the examination angle would be measured/defined with respect to one main mirror plane. The mirror 3 is configured to show the dental arch of the mouth where the frame 1 is placed. If the frame 1 is placed in the upper jaw, in particular in the vestibular space of the upper jaw or dental arch, the mirror 3 shows the upper dental arch. If the frame 1 is placed in the lower jaw, in particular in the vestibular space of the lower jaw or dental arch, the mirror 3 shows the lower dental arch.

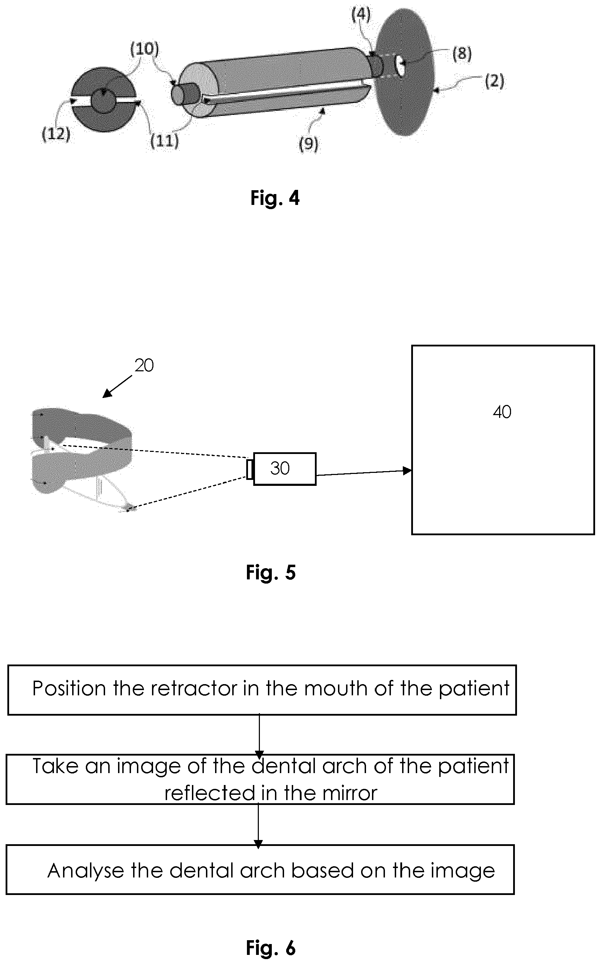

In a preferred embodiment, the mirror 3 can be moved between the examination state and at least one further state. The at least one further state could comprise a rest state, e.g. for transporting the retractor 20. The further state could comprise at least one further examination state. The at least one further examination state could correspond to at least one further examination angle (being different from the examination angle). The mirror 3 is preferably supported rotatably in the frame 1. Thus, the mirror 3 can be rotated around a rotation axis. The rotation axis is preferably parallel to the plane of the frame 1 and/or of the dental arch, when the frame 1 is positioned in the examination position. The rotation axis is preferably parallel to the throat side and/or close to the throat side of the mirror 3. The rotation axis of the mirror 3 is preferably arranged at the distal ends of the frame 1, preferably at a portion of the distal ends arranged in the active direction of the distal ends of the frame. Preferably, the distal ends of the frame 1 provide a protrusion 2 in the active direction, respectively, which are herein also called ears 2. Preferably, the rotation axis of the mirror 3 is arranged in these protrusions 2 or ears 2. This allows to arrange the mirror 3 also in the distal part of the dental arch above the teeth to provide a good view on the active side of the molar teeth of the dental arch. Preferably, the mirror 3 has two rotation pins 4 extending from the mirror 3 in the line of the rotation axis and being supported in two supports 8 in the distal ends of the frame 1, in particular in the ears 2 of the frame 1. However, it is also possible that the retractor 20 comprises an intermediate part as shown in Fig. 4 which is rotatably supported in the supports 8 in the frame 1 and the mirror 3 is fixed in a fixation. This solution will be described in more detail later.

Different rotation angles of the mirror 3 provide the different states of the mirror. The (at least one) examination state/angle is preferably configured to be fixed. Thus, the user could rotate the mirror 3 in the examination state/angle which is fixed so that the relative arrangement/angle of the mirror 3 with respect to the frame 1 does not change any more. This allows to introduce the retractor 20 in the examination state/angle in the examination position by holding (just) the mirror or (just) frame 1 without loosing the examination angle of the mirror 3. The fixation of the fixed examination angle could be achieved preferably by a mechanical mechanism. This could be a blocking mechanism activated by a user or a friction retention of the examination state/angle. The friction retention would require a certain force to put the mirror 3 in the examination state/angle and/or a certain force to release the mirror 3 from the examination state/angle. Once in the examination state/angle, the mirror 3 stays in the examination state/angle. The rest state is preferably substantially parallel to the plane of the frame 1. This allows to make the retractor 20 small and robust in the rest state and to put the mirror 3 only for its use in the examination state/angle. In one embodiment, the retractor 20 has two or more different examination angles. This could allow to use a smaller examination angles for patients which have maybe problems to open their mouth far enough for the ideal examination angle. Even if the patient is examined by another examination angle, the other examination angle is reproduceable as well. Even if it is preferred that the mirror 3 of the retractor 20 can be moved between different states/angles with respect to the frame 1, it would also be possible to realize the retractor 20 such that the mirror 3 cannot be moved between different states/angles and/or such that the mirror 3 has a fixed position with respect to the frame 1 (i.e. not rotatable). In this case, the mirror 3 and the frame 1 could be realised integrally made off one piece.

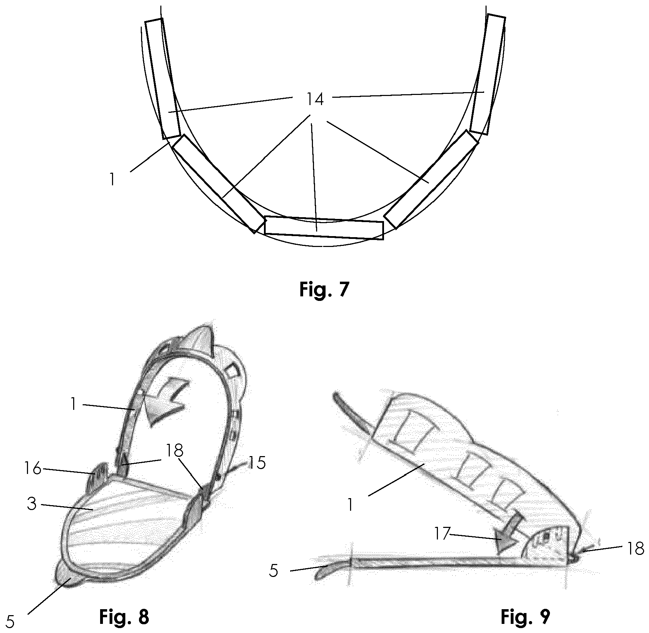

The embodiment of the retractor shown in Fig. 8 and 9 shows a mechanism to fix the mirror 3 and the frame 1 in the examination angle which could equally applied for the embodiment shown in Fig. 2 and 3. One of the frame 1 and mirror 3 comprises a male part 15, here a pin 15. The other of the frame 1 and the mirror 3 comprises at least one female part 16 configured to receive the male part 15, preferably holes 16 for receiving the pin 15. The at least one female part 16 comprises an examination female part 16. When the male part 15 is inserted into the examination female part 16, the mirror 3 is arranged in the examination state/angle with respect to the frame 1. This allows to fix the mirror 3 in the examination state/angle. The at least one female part 16 could comprise additional female parts 16 corresponding to different additional examination state/angles and/or to a rest angle/state. The embodiment of the retractor shown in Fig. 8 and 9 shows also the examination angle 17.

The mirror 3 or the reflective side of the mirror 3 is made of a reflective material. Preferably, the mirror 3 comprises on the reflective side a process treatment to make it reflective. Preferably, the surface treatment is a coating. Preferably, the mirror 3 is made of plastic with the mentioned surface treatment on the reflective side.

In a preferred embodiment, the retractor 20 comprises a secondary mirror 14. The secondary mirror 14 is arranged such that it reflects/projects the image of the facial side of the teeth on the mirror 3. Preferably, the secondary mirror 14 reflects the image of the facial side of all teeth of the dental arch on the mirror 3. However, it is also possible that the secondary mirror reflects the image of the facial side of (only) a sub-group teeth of the dental arch on the mirror 3, while the facial side of the remaining teeth of the dental arch might be visible directly through the mouth. The sub-group could comprise the molar and pre-molar teeth of the dental arch (and maybe as well the canine). Thus, the secondary mirror 14 allows that the mirror 3 shows an image of all three visible sides (lingual side, active side and facial side) of the teeth of the dental arch. This allows to see all visible surface of the teeth of the dental arch in only one image. The secondary mirror 14 is preferably arranged on the lingual side of the frame 1. Since the lingual side of the frame 1 faces the facial side of the dental arch in the examination position, the secondary mirror reflects/projects the image of the facial side of the teeth on the mirror 3. The mirror surface of the secondary mirror 14 extends thus substantially along the interdental direction and/or along (the vestibular side of) the dental arch. The mirror surface of the secondary mirror 14 is preferably inclined such that the image of the vestibular side of the teeth of the dental arch is reflected on the secondary mirror 14 on the mirror 3. The mirror surface of the secondary mirror 14 is preferably inclined so that the mirror surface extends from the lingual side of the frame 1 towards the facial side of the frame 1, when going in the active direction and/or so that the inclination direction of the mirror surface of the secondary mirror 14 is a superposition of the active direction and the facial direction. The secondary mirror 14 is preferably realized as a plurality of secondary sub-mirrors 14 as shown in Fig. 7. The secondary sub-mirrors 14 have preferably each a flat mirror surface so that the image of the facial side of the teeth is reflected without distortions on the mirror 3. A flat mirror surface means a mirror surface which is not curved. The secondary sub-mirrors 14 are preferably arranged so to extend along the frame 1 in the interdental direction and to form the secondary mirror 14 reflecting the complete image of the facial side of the teeth of the dental arch on the mirror 3. The secondary mirror 14 is or the secondary sub-mirrors 14 are realized preferably by a reflective coating or other reflective surface processing on the frame 1. The frame 1 is made preferably off plastic and the reflective surface of the secondary mirror 14 (or its sub-mirrors) is realized by a surface processing, preferably a coating. Preferably, the secondary mirror 14 (or its sub-mirrors) is arranged such that the image reflected from the secondary mirror 14 on the mirror 3 comprises the facial side of the teeth and the gums of the dental arch.

The previous description of the image of (the lingual side and the active side of) the teeth of the dental arch being reflected on the mirror 3 and/or the image of the facial side of the teeth of the dental arch being reflected over the secondary mirror 14 on the mirror 3 holds for the case that the reflector 20 or frame 1 is placed in the examination position, the mirror 3 is in the examination state/angle and/or the point of view for this image is looking from at least one viewpoint or view direction into the mouth of the patient having the retractor 20 in its mouth. The at least one view direction is preferably substantially perpendicular to the vertical direction of the head into the mouth of the patient. Depending on the examination angle and for the lower jaw, depending on the opening state of the mouth, the view direction for seeing the described image in the mirror 3 could also vary.

Preferably, the retractor 20 comprises a handle 5 for holding the retractor 20. The handle 5 is preferably arranged on the mirror 3. The handle 5 is preferably arranged on the mouth opening side of the mirror 3 so that the handle 5 extends is arranged outside of the mouth, when the retractor 20 is placed in the examination position. It is however also possible to arrange the handle 5 on the frame 1 or at any other part of the retractor 20. The user can hold the retractor 20 with the handle 5 and guide the retractor 20 in the examination position in the mouth. Since the mirror 3 in the examination state/angle has a stiff relationship with respect to the frame 1, the frame 1 of the retractor 20 can be brought in the examination position in the mouth by holding just the handle 5 of the mirror 3. Due to this stiff relationship, the user can maintain the retractor 20 or frame 1 in the examination position and keep the mirror 3 in the examination state/angle with only one hand holding the handle 5.

In the embodiment of Fig. 4, the retractor 20 comprises the intermediate part configured to hold the mirror 3 in the examination state/angle. Preferably, the mirror 3 can be removably fixed to the intermediate part. Here the fixation is a groove 11 in which one side of the mirror 3 is inserted. The fixation or groove 11 is preferably configured to hold the mirror 3 by friction. Thus, the retractor 20 with the frame 11 and the intermediate part can be used in combination with any intra-oral mirror as commercially available and common at dentists. The mirror can thus be disinfected after use and re-used with another retractor 20. The intermediate part could comprise two fixations for holding different types of mirrors, in particular mirrors of different thickness. A first fixation, preferably a first groove 11 is configured to hold a mirror of a first thickness and a second fixation, preferably a second groove 12 is configured to hold a mirror of a second thickness being larger than the first thickness. The first and second fixation/groove are preferably arranged with 180° angle difference on the intermediate part so that the fixed examination angle applies equally for both grooves. The intermediate part in the embodiment of Fig. 4 is realized by a rod 10, preferably made off plastic. A cylinder 9 is arranged around the rod 10. The rod 10 is a bit longer than the cylinder 9 so that the ends of the rod protrude from the ends of the cylinder 9 and provide rotation pins 4 to be inserted in the supports 8 of the frame. The first groove 11 and evtl. the second groove 12 are arranged in the cylinder 9. The cylinder 9 is preferably made of silicone. The elasticity of the silicone realises the friction fixation. This solution is further described below. The described solution with the intermediate part could also be used for a mirror 3 permanently fixed to the frame 11. In this case, the mirror 3 could be for example glued in the groove 11.

In one embodiment, the retractor 20 comprises a lighting system. The lighting system is configured to provide light in the mouth, when using the retractor 20 in the examination position and/or in the examination state/angle and/or when taking a photo of the dental arch in the mirror 3. The lighting system could be integrated in the frame 1, the mirror 3 or the intermediate part. The lighting system could be realized by a photoluminescent material like a fluorescent material. The photoluminescent material could save some energy which is emitted, when activated. Thus, the user would activate the lighting system before taking the photo of the dental arch. If the retractor 20 is a disposable, the activation mechanism could be a one-time mechanism. If the retractor 20 is for multiple uses, the activation mechanism could be rechargeable, e.g. by a heat over a certain time, e.g. by the heat from the steam for disinfecting the retractor 20 after use. Due to the emitted light from the lighting system, the quality of the photos would be improved. The lighting system could be arranged for example in the intermediate part.

The retractor 20 can be used in different examination positions. In the shown embodiment, the retractor 20 can be used in an upper examination position and in a lower examination position. In the upper examination position, the retractor 20 or frame 1 is placed in the upper jaw, preferably in the vestibular space of the upper jaw and/or is configured to show the dental arch of the upper jaw (upper dental arch) in the mirror 3. In the lower examination position, the retractor 20 or frame 1 is placed in the lower jaw, preferably in the vestibular space of the lower jaw and/or is configured to show the dental arch of the lower jaw (lower dental arch) in the mirror 3. The retractor 20 must just be turned upside down and can be placed in the respective other examination position to show the respective other dental arch. Thus, the retractor 20 allows in the upper examination position to take a photo of the upper dental arch and in the lower examination position to take a photo of the lower dental arch. It would be also possible that the retractor 20 has only one examination position in which it takes a photo of both dental arches in one view. This could be achieved for example by adding a second mirror for reflecting also the other dental arch and/or by fixing with the retractor 20 / frame 1 also the other jaw.

Preferably, the retractor 20 exists in different realisations with different sizes of the frame 1 corresponding to different sizes of dental arches. In one example, the retractor 20 is offered in three sizes large, medium and small to cover different anatomies of different patients. The retractor could be a disposable to be used just one time (for taking a photo of the upper and lower dental arch of a patient. However, it is also possible that the retractor 20 is designed to be used multiple times, e.g. in a dentist's office. In this case, the retractor must be sanitizable, e.g. by hot steam. The retractor 20 can also be designed for multiple use, but just for one and the same patient similar to a toothbrush.

Fig. 8 and 9 show another embodiment of the retractor 20. The embodiment corresponds mainly to the embodiment described previously, in particular with respect to Fig. 2 and 3. Instead of supporting the mirror 3 with rotation pins 4 in holes as supports, the rotation of the mirror 3 in the support of the frame is achieved by a bending line in the support. The mirror 3 and the frame 1 are thus manufactured out of one piece and the connection pieces 18 between the mirror 3 and the frame provide the supports with the bending line. The rest angle/state of the retractor could than be for example 180°, i.e. in the non-bended state of the retractor. The fixation of the examination angle is realised by a mechanism with a female part 16 and a male part 15 as already described before. Alternatively, the fixation of the examination angle could also be achieved by a stop position of the rotation movement between the frame 1 and the mirror 3 representing the examination angle. Thus, by pushing the retractor in the examination position in the mouth, the mirror 3 could be pushed into the examination angle or in the stop position. Thus, a reproducible examination angle can be achieved.

Fig. 5 shows a schematic embodiment of the system of the invention for taking a photographic image of the dental arch of a patient. The terms patient, person or human are here used interchangeable. The system comprises a retractor 20 as described previously, a camera 30 and preferably a processing means 40.

The camera 30 is configured to take a photographic image. Preferably, the camera 30 is a digital camera for taking a digital photographic image. The camera 30 is preferably connected over a network to the processing means 40. The network is preferably the internet. The camera 30 is preferably connected wirelessly to the network, e.g. by WLAN or a cellular phone network. The camera 30 is preferably a smartphone with a camera 30. The camera 30 or smartphone could have an application program for supporting the user taking the photo to hold the camera 30 and/or the retractor 20 correctly. The camera 30 is preferably configured to take an image of the dental arch of the patient, when the retractor 20 is in the examination position in the mouth of the patient and/or the mirror 3 is in the examination state/angle. The camera 30 is preferably held by a user. The user is preferably different than the patient. The user could be a dentist, a health assistant or any other third party. However, it is also possible that the user corresponds to the patient so that the patient takes the photo himself. Thus, the camera 30 is preferably independent from the retractor 30. However, it would also be possible to provide a fixed or reproduceable relationship between the retractor 20 and the camera 30. The retractor 20 could comprise the camera 30 itself or a holding means for holding the camera 30 at a determined reproduceable position. However, it is preferred to keep the camera 30 free from the retractor 20 so that the system is as easy and simple as possible. The process of taking the photographic image will be described in more detail below.

The processing means 40 is preferably a server. The server is preferably connected over the internet to the camera 30 to receive the image with the dental arch of the patient from the camera 30. The processing means 40 in a preferred embodiment is configured to analyse the dental arch of the patient based on the image with the dental arch of the patient received from the camera 30. This analysis could comprise the state of the teeth, the state of the gums, the absence of certain teeth, etc. The processing means 40 could use for example artificial intelligence to analyse the image. The processing means 40 is preferably configured to compare two images of the same dental arch of the same patient at different points in time to make conclusion about a certain development of the state of the dental arch.

Fig. 6 shows the steps of the method to take a photographic image (photo) of the dental arch of the patient.

In a first step, the retractor 20 is positioned in the mouth of the patient. Preferably, this involves putting the mirror 3 of the retractor 20 in the examination state/angle and/or to place the retractor 20 in the examination position in the mouth of the patient. Preferably, the user holds the retractor 20, preferably with its handle 5, in the examination position. Preferably, the user pushes the retractor 20 or its frame 1 in the vestibular space to obtain the examination position. However, in an alternative embodiment, it is also possible that the retractor 20 is kept in the examination position itself.

In a second step, a photo is taken of the dental arch of the patient reflected in the mirror 3 of the retractor 20 placed in the examination position in the mouth of the patient. Preferably, the user selects the position of the camera 30 such that the image of the camera 30 shows in the mirror 3 (the lingual side and the active side of) the dental arch and eventually shows also in the mirror 3 the facial side of the teeth reflected/projected over the secondary mirror 14 on the mirror 3. The system is so easy that a patient could also take the photo himself by holding the retractor 20 in the examination position with one hand and taking a selfie of the mirror 3 of the retractor 20 placed in the examination position with the other hand.

In a third step, the dental arch of the patient will be analysed based on the image. The analysis could comprise simply a dentist looking the image of the dental arch and making his conclusions. Preferably, the analysis comprises at least partly an analysis performed by the processing means 40. Preferably, the image taken in the second step is sent to the processing means 40 for performing the analysis. The processing means 40 could in a simple case show the image to a qualified person for doing the analysis. The processing means 40 could additionally perform some analysis in the processing means 40 to obtain some analysis results for supporting the analysis of the qualified person. The processing means 40 could also do the complete analysis alone to get some final conclusions.

Preferably, the three steps described are repeated for each dental arch of the patient. Thus, the retractor 20 is placed in the upper jaw of the patient in the upper examination position to obtain a photo of the upper dental arch and is placed in the lower jaw of the patient in the lower examination position to obtain a photo of the lower dental arch. Thus, a full dental analysis of the mouth of the patient can be done remotely by taking just two photos at the premises of the patient. Thus, elderly persons do not need to go to a dentist anymore and maybe also the intervals for the dentist for younger people could be extended as they could do remote visits by the described method. This method is very simple so that the photos can be taken by non-qualified persons. Nevertheless, the two photos of the upper and lower dental arch show all three visible sides of the teeth and gums of the dental arch. In addition, the palatine (for the photo of the upper dental arch) and the tongue (for the photo of the lower dental arch) of the patient are visible as well. Thus, a full dental analysis can be realised just based on the two photos. Due to the reproducibility of the examination positions, the conclusions derived from the photos have a higher quality. Since some analysis could be automatized at a high quality, the costs for the dental analysis could be significantly reduced. Since many health insurances do not cover dental care, this could make professional dental therapy accessible for a large part of the population for which the prices for the dentist's visit was prohibitive.

The described retractor 20, the system and the method could thus revolutionize the dental industry.

In the following, the retractor 20 is described again with different words without limiting the invention. The previous description and the subsequent description could be taken alone or in their combination.

The lip and cheek retractor 20 (Figure 2), proposed in the context of this invention, is more particularly intended for the oral examination of a patient, like a tongue depressor which helps to spread the cheeks or push back the tongue to better examine the inside of the oral cavity. Incorporating an intra-oral mirror 3 into the lip and cheek retractor 20 facilitates the oral examination since it allows a clear overall view of an entire dental arch, whether maxillary or mandibular, to be obtained without interference from the lips or cheeks, and eventually to take a photographic image of it. In contrast to most cheek and lip retractors 20, the retractor 20 according to the invention is fully inserted in the mouth, exactly like a dental impression tray. It will take its place in the entire vestibular space, i.e. for the upper or lower jaw in the space between the dental arches, the lips and the cheeks. The retractor 20 is articulated around a mirror held by a handle. It is through this handle that the operator can simultaneously orient the mirror and spread the lips and cheeks. By its shape, size and use, the retractor according to the invention could be less traumatic for older, physically more fragile patients.

The lip and cheek retractor 20 according to the invention is composed of two parts articulated between them (Figure 2). The first part described is the actual lip and cheek retractor 20, i.e. has the function to retract the lips and cheeks from the teeth of the dental arc. The first part is composed of a U-shaped or horseshoe frame 1 and follows the anatomy of the vestibular (external) alveolar processes of the upper and lower jaw. Like the impression trays used by the dentist to make impressions of the arches, the retractor can come in 3 sizes: small, medium and large, so as to cover the different anatomies of people.

Like the impression trays, the retractor 20 is preferably made of biocompatible plastic, single-use or autoclavable. The shape of the frame 1 is characterised by the anatomy of the dental arches since the free edge respects the lip brakes. In its posterior part, to the right and left, the frame 1 comprises a protrusion or ear 2. In the centre of the ear 2 is arranged a support 8. The support 8 is realised as a hole or recess. The support 8 or the whole runs preferably through its entire thickness of the frame 1. It is at the level of these supports 8 (one on each side of the frame 1) that the second part of the retractor 20 will be articulated or pivoted. The second part is a mirror 3. The mirror is preferably a single-use or autoclavable biocompatible plastic part that supports a reflective surface. The mirror 3 is articulated or pivoted in the supports 8 at the ears 2 of the frame 1, preferably by means of two lugs 4. The mirror 3 preferably follows the U-shape of the frame 1, as if to close its lower part (figure 2). At the end of the mirror 3 there is a gripping handle 5 which allows the operator to mobilize or hold the entire retractor. Indeed, this handle 5, which is attached to the body of the mirror 5, allows the angle of the mirror to be adjusted, as well as the degree of separation of the lips and cheeks, by a simple back and forth movement, from right to left.

In another version, the retractor 20 can be used with a commercially available intraoral mirror 7 as shown in Fig. 1. Mirrors for intraoral photography are commercially available in various shapes, made of polished metal or glass. In this case, the retractor 20 according to the invention will be composed of the frame 1 described above, the commercial intraoral mirror and a third part called intermediate part (figure 4) between the frame and the commercial intraoral mirror.

The so-called intermediate piece is a silicone cylinder 9 attached to a hard-plastic rod 10 which passes through it over its entire length. The length of the silicone cylinder 9 is the same as the posterior spacing of the frame 1 at the ears 2. The length of the plastic rod 10 exceeds that of the silicone cylinder 9, making the rotation pins 4 of this intermediate piece. These pins 4 are supported in the same support 8 provided in the centre of the ears 2 of the frame 1 of the retractor 20. The pins 4 are of the same diameter as the holes of the support 8, necessary for their rotation while maintaining a friction which in fact allows the mobilization of the whole spacer. A first groove 11 that runs the entire length of the silicone cylinder 9 allows friction fixation of the commercially available intraoral mirror. The commercially available mirror has its own gripping system. A second groove 12, diametrically opposite the first, also runs along the length of the cylinder, and differs from the first one by its width. This groove will be particularly used in the case of glass mirrors with an average thickness of 3 millimetres. Whereas the width of the first groove is relevant for polished steel mirrors with an average thickness of 1 millimetre. The cylinder 9 can be made of another material. The rod 10 can also be made of another material.

The device according to the invention shall preferably be made of a lightweight, single-use or autoclavable material. In another version, the spacer and the mirror (3) are connected by a friction rivet, allowing, however, the rotation of the mirror around the spacer. In another version, a miniaturized illumination system is located in the body of the silicone cylinder 9 between the frame 1 and the intraoral mirror.

A practical application of this oral mirror spacer according to the invention is the following. Upon admission of an elderly person to an institution, during the mandatory medical examination, the chief medical officer or nurse shall inquire about the patient's oral health, including whether the person wears dentures, or suffers from any particular oral problems. Increasingly, health authorities are asking for a "real" oral examination with a dental chart that shows missing, decayed or fractured teeth ... This is in order to assess the future resident's oral risks. The retractor 20 with the mirror proposed here, makes it possible to obtain an image of an entire dental arch in a single view, and moreover would allow to photo-graph this image using a smart phone. In two photo-graphic images, one for the upper and lower jaws, the examiner would obtain a "baseline oral status" of the patient at the time of the examination. Another advantage of obtaining an entire dental arch in a single photograph, without interference from the lips or cheeks, is that the image can be sent very quickly and easily over the Internet to a dentist. This photograph will be able to help the dentist to diagnose at a distance, especially for dependent people who cannot travel to the dental clinic.

It should be understood that the present invention is not limited to the described embodiments and that variations can be applied without going outside of the scope of the claims.

## Claims

1. A lip and cheek retractor for examining the oral cavity of a person, comprising:
- a U-shaped frame (1) which follows the form of a dental arch and comprising in its rear part on the right and on the left c support (8) and
- a mirror (3) being rotatable in the supports (8) of the frame (1)
**characterized in that**
the frame (1) is configured to be placed in the facial side space between a denta arch, a lip and cheeks of the oral cavity of the person such that the frame (1) follows the anatomy of a vestibular alveolar bone of the person.

2. Retractor according to claim 1, wherein the mirror (3) allows a view of an entire dental arch the person and/or allows to obtain an image of the entire dental arch in a single view.

3. Retractor according to one of the previous claims comprising a gripping handle (5), wherein the gripping handle (5) is arranged at the end of the mirror (3) and allows the operator to hold the lip and cheek retractor.

4. Retractor according to one of the previous claims configured to fix the mirror (3) in a certain rotation angle with respect to the frame (1), wherein the certain rotation angle is between 30° and 60° with respect to the plane of the frame.

5. Retractor according to one of the previous claims, wherein the frame (1) comprises a secondary mirror (14) arranged such that, when the frame (1) is placed in the mouth along the external alveolar bone of a dental arch of the person, a facial side of the dental arch is reflected via the secondary mirror (14) on the mirror (3).

6. Retractor according to one of the previous claims, wherein the supports are realised as a material connection between the frame (1) and the mirror (3) configured to allow a rotation of the mirror (3) in the supports of the frame (1).

7. A lip and cheek retractor for examining the oral cavity of a person, comprising:
- a U-shaped frame (1) which follows the form of a dental arch and comprising in its rear part on the right and on the left c support (8) and
- an intermediate part (9, 10) being rotatable in the supports (8) of the frame (1) and being configured to fix a mirror (3)
**characterized in that**
the frame (1) is configured to be placed in the facial side space between a denta arch, a lip and cheeks of the oral cavity of the person such that the frame (1) follows the anatomy of a vestibular alveolar bone of the person.

8. Retractor according to the previous claim, wherein the intermediate part comprises a cylinder (9) and a rod (10), said cylinder (9) being secured to the rod (10) which passes through it over its entire length, said rod (10) exceeding in length that of the cylinder (9) and thus forming rotation pins (4) which are supported in the supports (8) provided in the frame(1), the cylinder (9) being configured to fix the mirror (3).

9. Retractor according to claim 7 or 8, **characterized in that** the intermediate part or the cylinder (9) comprises a groove (11) running over the entire length of the intermediate part or the cylinder (9) for fixing the mirror (3) by friction.

10. System for taking a photographic image of a dental arch of a person comprising
either a retractor (20) according to one of claims 7 to 9 with a mirror (3) fixed in the intermediate part (9, 10) or a retractor (20) according to one of claims 1 to 6; and
a camera (30) for taking a photographic image of the mirror (3) showing the dental arch of the person, when the retractor (20) is placed in the oral cavity of the person.

11. System according to the previous claim comprising further a processing means (40) configured to receive the photographic image from the camera (30) and to analyse the dental arch of the person based on the photographic image.

12. System according to claim 11, wherein the analysis of the dental arch comprises the detection of at least one health problem of the dental arch, wherein the at least one health problem is detected in the image based on an artificial intelligence engine of the processing means (40).

13. System according to the previous claim, wherein the camera (30) is a smartphone with a camera.

14. Method for taking a photographic image of a dental arch of a person comprising the steps:
placing a retractor (20) into the oral cavity of the person, wherein the retractor (20) being either a retractor according to one of claims 7 to 9 with a mirror (3) fixed in the intermediate part (9, 10) or a retractor according to one of claims 1 to 6; and
using a camera (30) to take a photographic image of the mirror (3) showing the dental arch of the person, when the retractor (20) is placed in the oral cavity of the person.

15. Method according to claim 14 comprising the steps of:
receiving the image with the dental arch of the patient from the camera (30) at a processing means (40), wherein the processing means (40) is a server connected over the internet to the camera (30);
analysing, in the processing means (40), the dental arch of the person based on the photographic image, wherein the analysis of the dental arch comprises the detection of at least one health problem of the dental arch, wherein the at least one health problem is detected in the image based on an artificial intelligence engine of the processing means (40).

## Patentansprüche

1. Lippen- und Wangenretraktor zum Untersuchen der Mundhöhle einer Person, der Folgendes umfasst:
- einen U-förmigen Rahmen (1), der der Form eines Zahnbogens folgt und in seinem hinteren Teil rechts und links einen
Träger (8) umfasst und
- einen Spiegel (3), der in den Trägern (8) des Rahmens (1) drehbar ist,
**dadurch gekennzeichnet, dass** der Rahmen (1) dazu konfiguriert ist, in der in dem Gesichtsseitenraum zwischen einem Zahnbogen, einer Lippe und Wangen der Mundhöhle der Person derart platziert zu sein, dass der Rahmen (1) der Anatomie eines vestibulären Alveolarknochens der Person folgt.

2. Retraktor nach Anspruch 1, wobei der Spiegel (3) eine Sicht eines gesamten Zahnbogens der Person erlaubt, und/oder es erlaubt, ein Bild des gesamten Zahnbogens in einer einzigen Sicht zu erhalten.

3. Retraktor nach einem der vorstehenden Ansprüche, der einen Handgriff (5) umfasst, wobei der Handgriff (5) an dem Ende des Spiegels (3) eingerichtet ist und es dem Bediener erlaubt, den Lippen- und Wangenretraktor zu halten.

4. Retraktor nach einem der vorstehenden Ansprüche, der dazu konfiguriert ist, den Spiegel (3) in einem bestimmten Rotationswinkel in Bezug auf den Rahmen (1) zu fixieren, wobei der bestimmte Rotationswinkel zwischen 30° und 60° in Bezug auf die Ebene des Rahmens liegt.

5. Retraktor nach einem der vorstehenden Ansprüche, wobei der Rahmen (1) einen sekundären Spiegel (14) umfasst, der derart eingerichtet ist, dass, wenn der Rahmen (1) in dem Mund entlang des externen Alveolarknochens eines Zahnbogens der Person platziert ist, eine Gesichtsseite des Zahnbogens über den sekundären Spiegel (14) auf dem Spiegel (3) reflektiert wird.

6. Retraktor nach einem der vorstehenden Ansprüche, wobei die Träger als eine Materialverbindung zwischen dem Rahmen (1) und dem Spiegel (3) hergestellt ist, die dazu konfiguriert ist, eine Drehung des Spiegels (3) in den Trägern des Rahmens (1) zu erlauben.

7. Lippen- und Wangenretraktor zum Untersuchen der Mundhöhle einer Person, der Folgendes umfasst:
- einen U-förmigen Rahmen (1), der der Form eines Zahnbogens folgt und in seinem hinteren Teil rechts und links einen Träger (8) umfasst und
- ein Zwischenteil (9, 10), das in den Trägern (8) des Rahmens (1) drehbar und dazu konfiguriert ist, einen Spiegel (3) zu fixieren,
**dadurch gekennzeichnet, dass** der Rahmen (1) dazu konfiguriert ist, in der in dem Gesichtsseitenraum zwischen einem Zahnbogen, einer Lippe und Wangen der Mundhöhle der Person derart platziert zu sein, dass der Rahmen (1) der Anatomie eines vestibulären Alveolarknochens der Person folgt.

8. Retraktor nach dem vorstehenden Anspruch, wobei das Zwischenteil einen Zylinder (9) und eine Stange (10) umfasst, wobei der Zylinder (9) an der Stange (10), die durch ihn über seine gesamte Länge durchgeht, befestigt ist, wobei die Stange (10) in der Länge die des Zylinders (9) überschreitet und daher Drehstifte (4) bildet, die in den Trägern (8), die in dem Rahmen (1) bereitgestellt sind, getragen sind, wobei der Zylinder (9) dazu konfiguriert ist, den Spiegel (3) zu fixieren.

9. Retraktor nach Anspruch 7 oder 8, **dadurch gekennzeichnet, dass** das Zwischenteil oder der Zylinder (9) eine Nut (11) umfasst, die über die gesamte Länge des Zwischenteils oder des Zylinders (9) zum reibschlüssigen Fixieren des Spiegels (3) verläuft.

10. System zum Aufnehmen eines fotografischen Bildes eines Zahnbogens einer Person, das
entweder einen Retraktor (20) nach einem der Ansprüche 7 bis 9 mit einem Spiegel (3), der in den Zwischenteil (9, 10) fixiert ist, oder einen Retraktor (20) nach einem der Ansprüche 1 bis 6 umfasst; und
eine Kamera (30) zum Aufnehmen eines fotografischen Bildes des Spiegels (3), das den Zahnbogen der Personen zeigt, wenn der Retraktor (20) in der Mundhöhle der Person platziert ist.

11. System nach dem vorstehenden Anspruch, das weiter ein Verarbeitungsmittel (40) umfasst, das dazu konfiguriert ist, das fotografische Bild von der Kamera (30) zu empfangen und den Zahnbogen der Person basierend auf dem fotografischen Bild zu analysieren.

12. System nach Anspruch 11, wobei die Analyse des Zahnbogens die Erfassung mindestens eines Gesundheitsproblems des Zahnbogens umfasst, wobei das mindestens eine Gesundheitsproblem in dem Bild basierend auf einer Maschine mit künstlicher Intelligenz des Verarbeitungsmittels (40) erfasst wird.

13. System nach dem vorstehenden Anspruch, wobei die Kamera (30) ein Smartphone mit einer Kamera ist.

14. Verfahren zum Aufnehmen eines fotografischen Bildes eines Zahnbogens einer Person, das die folgenden Schritte umfasst:
Platzieren eines Retraktor (20) in der Mundhöhle der Person, wobei der Retraktor (20) entweder ein Retraktor nach einem der Ansprüche 7 bis 9 mit einem Spiegel (3), der in dem Zwischenteil (9, 10) befestigt ist, oder ein Retraktor nach einem der Ansprüche 1 bis 6 ist; und
Verwenden einer Kamera (30) zum Aufnehmen eines fotografischen Bildes des Spiegels (3), das den Zahnbogen der Personen zeigt, wenn der Retraktor (20) in der Mundhöhle der Person platziert ist.

15. Verfahren nach Anspruch 14, das die folgenden Schritte umfasst:
Empfangen des Bildes mit dem Zahnbogen des Patienten von der Kamera (30) an einem Verarbeitungsmittel (40), wobei das Verarbeitungsmittel (40) ein Server ist, der über das Internet mit der Kamera (30) verbunden ist;
Analysieren in dem Verarbeitungsmittel (40) des Zahnbogens der Person basierend auf dem fotografischen Bild, wobei die Analyse des Zahnbogens die Erfassung mindestens eines Gesundheitsproblems des Zahnbogens umfasst, wobei das mindestens eine Gesundheitsproblem in dem Bild basierend auf einer Maschine mit künstlicher Intelligenz des Verarbeitungsmittels (40) erfasst wird.

## Revendications

1. Écarteur de lèvres et de joues pour examiner la cavité buccale d'une personne, comprenant :
- un cadre en forme de U (1) qui suit la forme d'une arcade dentaire et comprenant dans sa partie arrière sur la droite et sur la gauche un support (8) et
- un miroir (3) qui peut tourner dans les supports (8) du cadre (1),
**caractérisé en ce que** le cadre (1) est configuré pour être placé dans l'espace latéral du visage entre une arcade dentaire, une lèvre et les joues de la cavité buccale de la personne de telle sorte que le cadre (1) suive l'anatomie d'un os alvéolaire vestibulaire de la personne.

2. Écarteur selon la revendication 1, dans lequel le miroir (3) permet une visualisation de toute une arcade dentaire de la personne et/ou permet d'obtenir une image de toute l'arcade dentaire dans une seule vue.

3. Écarteur selon l'une des revendications précédentes comprenant une poignée de préhension (5), dans lequel la poignée de préhension (5) est agencée au niveau de l'extrémité du miroir (3) et permet à l'opérateur de tenir l'écarteur de lèvres et de joues.

4. Écarteur selon l'une des revendications précédentes configuré pour fixer le miroir (3) dans un certain angle de rotation par rapport au cadre (1), dans lequel le certain angle de rotation est entre 30° et 60° par rapport au plan du cadre.

5. Écarteur selon l'une des revendications précédentes, dans lequel le cadre (1) comprend un miroir secondaire (14) agencé de telle sorte que, lorsque le cadre (1) est placé dans la bouche le long de l'os alvéolaire vestibulaire d'une arcade dentaire de la personne, un côté du visage de l'arcade dentaire soit réfléchi par le biais du miroir secondaire (14) sur le miroir (3).

6. Écarteur selon l'une des revendications précédentes, dans lequel les supports sont réalisés sous la forme d'une connexion de matériel entre le cadre (1) et le miroir (3) configurée pour permettre une rotation du miroir (3) dans les supports du cadre (1).

7. Écarteur de lèvres et de joues pour examiner la cavité buccale d'une personne, comprenant :
- un cadre en forme de U (1) qui suit la forme d'une arcade dentaire et comprenant dans sa partie arrière sur la droite et sur la gauche un support (8) et
- une partie intermédiaire (9, 10) qui peut tourner dans les supports (8) du cadre (1) et qui est configurée pour fixer un miroir (3),
**caractérisé en ce que** le cadre (1) est configuré pour être placé dans l'espace latéral du visage entre une arcade dentaire, une lèvre et les joues de la cavité buccale de la personne de telle sorte que le cadre (1) suive l'anatomie d'un os alvéolaire vestibulaire de la personne.

8. Écarteur selon la revendication précédente, dans lequel la partie intermédiaire comprend un cylindre (9) et une tige (10), ledit cylindre (9) étant fixé à la tige (10) qui passe à travers celui-ci sur toute sa longueur, ladite tige (10) dépassant en longueur celle du cylindre (9) et, de ce fait, formant des broches de rotation (4) qui sont supportées dans les supports (8) disposés dans le cadre (1), le cylindre (9) étant configuré pour fixer le miroir (3).

9. Écarteur selon la revendication 7 ou 8, **caractérisé en ce que** la partie intermédiaire ou le cylindre (9) comprend une rainure (11) s'étendant sur toute la longueur de la partie intermédiaire ou le cylindre (9) pour fixer le miroir (3) par friction.

10. Système pour prendre une image photographique d'une arcade dentaire d'une personne comprenant
soit un écarteur (20) selon l'une des revendications 7 à 9 avec un miroir (3) fixé dans la partie intermédiaire (9, 10), soit un écarteur (20) selon l'une des revendications 1 à et
un appareil photographique (30) pour prendre une image photographique du miroir (3) montrant l'arcade dentaire de la personne lorsque l'écarteur (20) est placé dans la cavité buccale de la personne.

11. Système selon la revendication précédente, comprenant en outre un moyen de traitement (40) configuré pour recevoir l'image photographique en provenance de l'appareil photographique (30) et pour analyser l'arcade dentaire de la personne sur la base de l'image photographique.

12. Système selon la revendication 11, dans lequel l'analyse de l'arcade dentaire comprend la détection d'au moins un problème de santé de l'arcade dentaire, dans lequel le au moins un problème de santé est détecté dans l'image sur la base d'un moteur d'intelligence artificielle du moyen de traitement (40).

13. Système selon la revendication précédente, dans lequel l'appareil photographique (30) est un téléphone intelligent présentant un appareil photographique.

14. Procédé pour prendre une image photographique d'une arcade dentaire d'une personne comprenant les étapes consistant à :
placer un écarteur (20) dans la cavité buccale de la personne, dans lequel l'écarteur (20) est soit un écarteur selon l'une des revendications 7 à 9 avec un miroir (3) fixé dans la partie intermédiaire (9, 10), soit un écarteur selon l'une des revendications 1 à et
utiliser un appareil photographique (30) pour prendre une image photographique du miroir (3) montrant l'arcade dentaire de la personne lorsque l'écarteur (20) est placé dans la cavité buccale de la personne.

15. Procédé selon la revendication 14 comprenant les étapes consistant à :
recevoir l'image avec l'arcade dentaire du patient en provenance de l'appareil photographique (30) au niveau d'un moyen de traitement (40), dans lequel le moyen de traitement (40) est un serveur connecté à l'internet à l'appareil photographique (30) ;
analyser, dans le moyen de traitement (40), l'arcade dentaire de la personne sur la base de l'image photographique, dans lequel l'analyse de l'arcade dentaire comprend la détection d'au moins un problème de santé de l'arcade dentaire, dans lequel le au moins un problème de santé est détecté dans l'image sur la base d'un moteur d'intelligence artificielle du moyen de traitement (40).
